(19) Europäisches Patentamt
European Patent Office
Office européen des brevets

(11) Publication number: 0 129 059 B1

(12) EUROPEAN PATENT SPECIFICATION

(45) Date of publication of patent specification: 28.01.87

(51) Int. Cl.$^4$: C 07 D 263/32, G 03 G 5/06

(21) Application number: 84105515.5

(22) Date of filing: 15.05.84

# (54) Oxazole derivatives, process for production thereof and use thereof as photoconductive material.

(30) Priority: 19.05.83 JP 88233/83

(43) Date of publication of application: 27.12.84 Bulletin 84/52

(45) Publication of the grant of the patent: 28.01.87 Bulletin 87/05

(84) Designated Contracting States: DE GB

(56) References cited: DE-C-1 120 875

(73) Proprietor: Hitachi Chemical Co., Ltd.
1-1, Nishi-shinjuku 2-chome Shinjuku-ku
Tokyo 160 (JP)

(72) Inventor: Hayashida, Shigeru
16-5-402, Sukegawacho-5-chome
Hitachi-shi (JP)

(74) Representative: Kraus, Walter, Dr. et al
Patentanwälte Kraus, Weisert & Partner
Thomas-Wimmer-Ring 15
D-8000 München 22 (DE)

EP 0 129 059 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to oxazole derivatives, a process for production thereof and use thereof as photo-conductive materials.

As oxazole derivatives, there have heretofore been known 2-(4-dipropylaminophenyl)-4-(4-dimethylaminophenyl)-5-(2-chlorophenyl)-1,3-oxazole [compound (IV)], 2-(4-diethylaminophenyl)-4-(4-dimethylaminophenyl)-5-(2-chlorophenyl)-1,3-oxazole [compound (V)] 2-(4-dimethylaminophenyl)-4-(4-dimethylaminophenyl)-5-(2-chlorophenyl)-1,3-oxazole [compound (VI)] and the like, and they are utilized as electrophotographic photosensitive materials for copying machines and laser beam printers (Japanese Patent Appln. Kokia (Laid-Open) No. 123544/81). However, these oxazole derivatives are disadvantageous in that since they are easily decomposed in solution by ultraviolet light to mainly give 2-(4-dipropylaminophenyl)-6-dimethylaminophenanthro[9,10-d]oxazole as a decomposition product, they require care in handling and are apt to lose their characteristics during production of an electrophotographic plate.

This invention aims at solving these problems and providing an oxazole derivative which is stable to ultraviolet light and useful as an electrophotographic photosensitive material or electrochromism display materials, a process for producing the same and a process for using the same.

This invention provides an oxazole derivative represented by the formula:

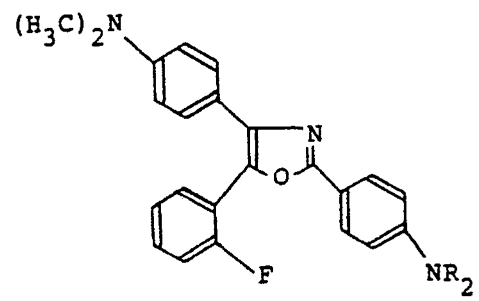

(I)

wherein R is an alkyl group having 1 to 3 carbon atoms.

This invention further provides a process for producing an oxazole derivative of the formula (I) by reacting a compound represented by the formula:

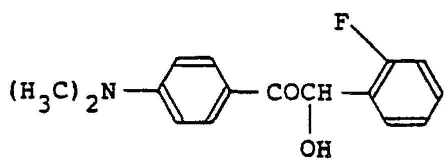

(II)

with a compound represented by the formula:

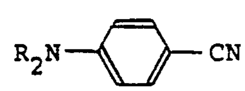

(III)

wherein R is an alkyl group having 1 to 3 carbon atoms.

This invention still further provides a process for using the oxazole derivative of the formula (I) as a photoconductive material.

In the attached drawings, Fig. 1 is an NMR spectrum of the oxazole derivative obtained in Example 1, Fig. 2 is an Ir spectrum thereof, Fig. 3 is an NMR spectrum of the oxazole derivative obtained in Example 2, Fig. 4 is an IR spectrum thereof, Fig. 5 is an NMR spectrum of the oxazole derivative obtained in Example 3, Fig. 6 is an IR spectrum thereof, Fig. 7 is an NMR spectrum of the oxazole derivative obtained in the Comparative Example, Fig. 8 is an IR spectrum thereof, and Fig. 9 is the changes with the lapse of time in the rates of decomposition of respective acetonitrile solutions of the compounds (VIII), (IX), (X) and (V), by irradiation from a high pressure mercury arc lamp.

The oxazole derivative represented by

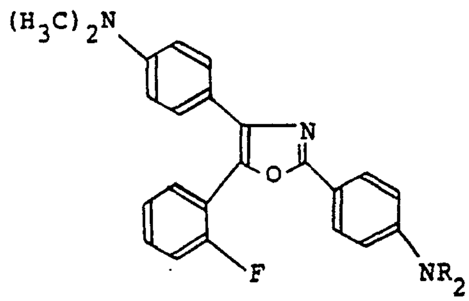

(I)

wherein R is preferably a methyl group, an ethyl group or a propyl group, is a novel compound, and has a F atom in place of the Cl atom in each of the well-known compounds (IV), (V) and (VI); therefore it is so stable to ultraviolet light that it is not or difficult to be decomposed by irridiation with ultraviolet light.

The oxazole derivative of the formula (I) can be produced by reacting a compound represented by the formula:

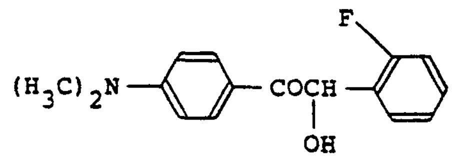

(II)

with a compound represented by the formula:

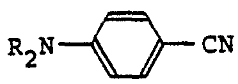

(III)

wherein R is preferably a methyl group, an ethyl group or a propyl group.

The compound represented by the formula (II) can be produced, for example, by adding, to a 50% aqueous ethanol solution, 4-dimethylaminobenzaldehyde, 2-fluoro-benzaldehyde and KCN in proportions of 1 mole, 1 mole and 0.1 to 1.5 moles, respectively, and refluxing the resulting mixture for 1.5 to 4 hours.

The compound represented by the formula (III) can be synthesized, for example, by the following reaction path. One mole of aniline is reacted with 0.67 mole of a compound represented by the formula:

$(RO)_3PO$ (VII)

wherein R is preferably a methyl group, an ethyl group or a propyl group, under reflux for 2 hours, and the reaction solution is distilled under reduced pressure to obtain a dialkylaniline (in which the alkyl group is a methyl group, an ethyl group or a propyl group; hereinafter the same applies). Subsequently, 0.3 to 0.4 mole of the dialkylaniline is added dropwise to 1 mole of N,N-dimethylformamide at 19° to 22°C, and after completion of the addition, the resulting mixture is heated at 90°C to 95°C for 2 hours, after which the reaction solution thus obtained is distilled under reduced pressure to obtain a dialkylaminobenzaldehyde. With 1 mole of the dialkylaminobenzaldehyde is reacted 1.0 to 1.3 moles of hydroxylamine hydrochloride at room temperature for 20 minutes, and the reaction solution is allowed to stand in a refrigerator to deposit a dialkylaminobenzaldehyde oxime. Finally, 1 mole of the dialkylaminobenzaldehyde oxime is reacted with 2 to 3 moles of acetic anhydride under heating and reflux for 20 minutes to be dehydrated, whereby the compound represented by the formula (III) can be produced.

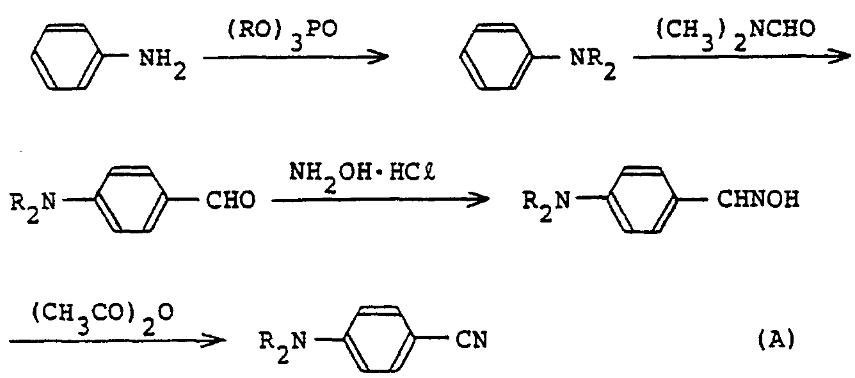


(A)

In the above, production of the dialkylaminobenzonitrile from the dialkylaminobenzaldehyde can be carried out also by the following reaction part (B).

With 1 mole of the dialkylaminobenzaldehyde are mixed 1.15 moles of $NH_2OH$-HCl, a large excess of HCOONa and about 1,500 ml of formic acid, and the resulting mixture is subjected to reaction under reflux for 1 hour, immediately after which the reaction solution thus obtained is poured into water to deposit a precipitate of the dialkylaminobenzonitrile. It is sufficient that the precipitate is recrystallized from water.

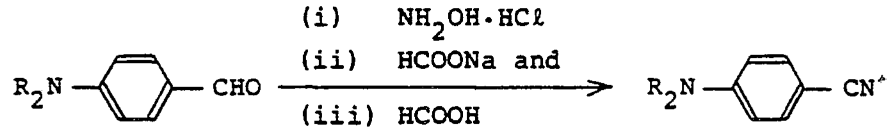


(B)

The oxazole derivative represented by the formula (I) can be obtained by mixing well the compound of the formulae (II) and (III) and then adding the resulting mixture to concentrated sulfuric acid with stirring in the dark. The reaction temperature is preferably 20° to 70°C. When the reaction temperature is adjusted to 50° to 70°C, the reaction is completed in 0.5 to 2 hours. The reaction product can be purified by recrystallization from methanol.

It is preferable to mix the compounds of the formulae (II) and (III) previously before the reaction. When the compound of the formula (II) alone is added to concentrated sulfuric acid, it undergoes dehydration reaction before reacting with the compound represented by the formula (III).

The oxazole derivative represented by the formula (I) can effectively be used as a photoconductive material. This photoconductive material can be used as a charge transport material in an electrophotographic plate by taking advantage of its photoconductivity.

When an electrophotographic plate is produced by using the oxazole devivative of the formula (I) of this invention as a charge transport material for a material for electrophotography, a monolayer comprising a mixture of the compound represented by the formula (I) and a charge generating material can be formed as a photoconductive layer on an electroconductive layer. It is also possible to form a so-called double layer structure as a photoconductive layer on an electroconductive layer by forming individual layers of a charge generating material and a charge transport material.

When the monolayer structure is employed, the proportion of the aforesaid charge transport material to the charge generating material is usually 1 to 50 parts by weight of the former to 1 part by weight of the latter. The thickness of the whole photoconductive layer is usually adjusted to 5 to 100 µm. On the other hand, when the double layer structure is employed, it is usual to adjust the thickness of the charge generating material layer to 0.1 to 5 µm and the thickness of the charge transport material layer to 5 to 100µm. However, it is desirable in either case to determine them finally with caution so as not to impair the photosensitivity, i.e., the electrification characteristics. When the thickness of the photoconductive layer is too large, the flexibility of the layer itself tends to be lowered, and therefore care must be taken.

The aforesaid charge generating material includes Si, Se, $AS_2S_3$, $Sb_2S_3$, $Sb_2Se_3$, CdS, CdSe, CdTe, ZnO, phthalocynine pigments, azo pigments anthraquinone pigments, indigoid pigments, quinacridone pigments, perylene pigments, polycyclic quinone pigments, methine squalate pigments, dyes, etc. Already-known binding agents may be used in the photoconductive layer. The binding agent is selected from linear saturated polyester resins, polycarbonate resins, acrylic resins, butyral resins, polyketone resins, polyurethane resins, poly-N-vinyl-carbazole, poly(p-vinylphenyl)anthracene and the like. The amount of the binding agent used is suitably 1 to 50 parts by weight per 1 part by weight of the charge generating material in the case of a photoconductor having a monolayer structure. In the case of a ohotoconductor having a double layer structure, the binding agent should be added to at least the layer comprising a charge transport material, and in this case, the amount of the binding agent is suitably 1 to 50 parts by weight per 1 part by weight of the charge transport material. In the electroconductive layer, aluminium, brass, copper, gold or the like is used.

This invention is explained below in detail referring to examples.

Synthesis Example 1

[Production of 4-dimethylamino-2'-fluorobenzoin]

In 150 ml of a 50% aqueous ethanol solution were dissolved 14.9 g of dimethylaminobenzaldehyde, 12.4 g of 2-fluorobenzaldehyde and 8.0 g of potassium cyanide, and the resulting solution was heated and refluxed for 2 hours and 30 minutes. After the heating and refluxing, the solution was allowed to cool, and the light-yellow crystals deposited were filtered and then dried at 80°C. The crystals were recrystallized from 500 ml of methanol to obtain 4-dimethylamino-2'-fluorobenzoin (yield 45%) having a melting point of 168°—170°C.

Synthesis Example 2

[Production of 4-dipropylaminobenzonitrile]

After 62 g of aniline and 100 g of n-propyl phosphate were heated and refluxed for 2 hours, the resulting solution was allowed to cool, and when its temperature was lowered to 55° to 60°C, an aqueous sodium hydroxide solution (56.3 g/225 ml) was added, and the resulting solution was heated and refluxed for 1 hour. Immmediately after completion of the heating and reflux, the reaction solution was transferred

into a 1-liter beaker and allowed to stand. The reaction solution separated into two layers, and the upper layer was yellow, while the lower layer became a white solid. The white solid was extracted with three 100-ml portions of ether, and the upper layer and the combined extract were dried over anhydrous sodium sulfate. After the ether was distilled off, the same volume of acetic anhydride was added, and the resulting solution was allowed to stand overnight. An aqueous hydrochloric acid solution (45 ml/67.5 ml) was added to this reaction solution and the resulting mixture was allowed to cool, and thereafter a 25% aqueous sodium hydroxide solution was added, upon which the mixture separated into an oil layer and an aqueous layer. The aqueous layer was extracted with three 100-ml portions of ether, and the combined extract was dried together with the oil layer over anhydrous sodium sulfate. After the ether was distilled off, the residue was distilled under reduced pressure (b.p. 130°—148°C/18 mmHg) to obtain dipropylamiline (yield 69%).

Next, 44.5 g of phosphorus oxychloride was added dropwise to 54 g of N,N-dimethylformamide over a period of 30 minutes while the N,N-dimethylformamide was maintained at 20°C or lower.

Thereto was added dropwise 35 g of the dipropylaniline obtained in the above over a period of 1 hour. After completion of the addition, the resulting mixture was stirred with heating at 90° to 95°C for 2 hours. Thereafter, the reaction solution thus obtained was added to ice water, and the resulting mixture was neutralized with a saturated aqueous sodium acetate solution. The mixture was then extracted with five 100-ml portions of ether and the combined extract was dried over anhydrous sodium sulfate. After the ether was distilled off, the residue was distilled under reduced pressure (b.p. 155°—163°C/3 mmHg) to obtain dipropylaminobenzaldehyde (yield 46%).

In 23 ml of 95% ethanol was dissolved 11.6 g of the dipropylaminobenzaldehyde, and an aqueous hydroxylamine hydrochloride solution (4.7 g/5.7 ml) was added thereto, after which the resulting solution was stirred for 20 minutes. An aqueous sodium hydroxide solution (3.4 g/4.5 ml) was added to the reaction solution, and the resulting mixture was allowed to stand at room temperature for 2 hours and 30 minutes. Thereto was added 29 g of ice and the air in the reaction system was replaced with $CO_2$ gas, after which the reaction system was allowed to stand overnight in a refrigerator. The white crystals deposited was filtered by means of suction washed with water, and then dried.

To the compound thus obtained was added 12.4 g of acetic anhydride, and the resulting mixture was heated and refluxed for 20 minutes. Thereafter, 35 ml of cold water was added to the reaction solution thus obtained, and the resulting solution was neutralized with an aqueous sodium hydroxide solution. The solution neutralized was extracted with three 40-ml portions of ether, and the combined extract was dried over anhydrous sodium sulfate. After the ether was distilled off, the residue was distilled under reduced pressure (b.p. 180°C/4 mm Hg) to obtain 4-dipropylaminobenzonitrile (yield 79%). When allowed to stand, this compound crystallized into yellow crystals having a melting point of 39°—41°C.

## Synthesis Example 3

[Production of 4-dimethylaminobenzonitrile]

There were heated and refluxed for 1 hour 29.8 g of dimethylaminobenzaldehyde synthesized according to the procedure in Synthesis Example 2, 15.9 g of hydroxylamine hydrochloride, 25 g of sodium formate and 333 ml of formic acid. When the reaction solution was added dropwise to 800 ml of water with stirring, light-green crystals were deposited. The crystals were filtered, thereafter dried, and then recrystallized from acetone to obtain 4-dimethylaminobenzonitrile (yield 50%) having a melting point of 67°—69°C.

## Synthesis Example 4

[Production of 4-diethylaminobenzonitrile]

There were heated and refluxed for 1 hour 25 g of diethylaminobenzaldehyde synthesized according to the procedure in Synthesis Example 2, 10.5 g of hydroxylamine hydrochloride, 16.5 g of sodium formate and 220 ml of formic acid. When the reaction solution was added dropwise to 400 ml of water with stirring, white crystals were deposited. The crystals were filtered, thereafter dried, and then recrystallized from water-acetone to obtain 4-diethylaminobenzonitrile (yield 50%) having a melting point of 66°—67°C.

## Example 1

In the dark, 35 g of concentrated sulfuric acid was maintained at 60°C and a mixture of 2.4 g of 4-dimethylamino-2′-fluorobenzoin obtained in Synthesis Example 1 and 2.1 g of the 4-dipropylaminobenzonitrile obtained in Synthesis Example 2 was added, after which the resulting mixture was stirred for 1 hour. When the reaction solution thus obtained was added to 900 ml of water, yellowish-green crystals were deposited. After the crystals were filtered, the filtrate was made basic with a 3N aqueous sodium hydroxide solution, upon which light-yellow crystals were deposited. The crystals were filtered by means of suction and air-dried. The crystals were then recrystallized from methanol to obtain 1.73 g (yield 43%) of a light-yellow solid. The light-yellow solid was identified as 2-(4-dipropylaminophenyl)-4-(4-dimethylaminophenyl)-5-(2-fluorophenyl)-1,3-oxazole (the compound VIII) from the following analysis results

(1) Melting point 134°—135°C

(2) Elementary analysis values: $C_{29}H_{32}N_3OF$

| | C | H | N |
|---|---|---|---|
| Calculated (%) | 76.15 | 7.00 | 9.19 |
| Found (%) | 76.07 | 7.11 | 9.15 |

(3) NMR values (the NMR spectrum is shown in Fig. 1): $CDCl_3$

τ values

| | | |
|---|---|---|
| 2.07 | (doublet (2H) | aromatic ring |
| 2.38—2.82 | (multiplet 6H) | |
| 3.37 | (doublet 2H) | |
| 3.40 | (doublet 2H) | |

6.72 (triplet 4H the methylene proton of a propyl group)

7.11 (singlet 6H the proton of a methyl group)

8.46 (sextet 4H the central methylene proton of a propyl group)

9.08 (triplet 6H the terminal methyl proton of a propyl group)

(4) IR spectrum (KBr method) is shown in Fig. 2.

## Example 2

In the dark, 35 g of concentrated sulfuric acid was maintained at 60°C and a mixture of 2.4 g of the 4-dimethylamino-2'-fluorobenzoin obtained in Synthesis Example 1 and 1.5 g of the 4-dimethylaminobenzonitrile obtained in Synthesis Example 3 was added, after which the resulting mixture was stirred for 30 minutes. When the reaction solution thus obtained was added to 900 ml of water, yellowish-green crystals were deposited. After the crystals were filtered, the filtrate was made basic with a 3N aqueous sodium hydroxide solution, upon which light-yellow crystals were deposited. The crystals were filtered by means of suction and air-dried. The crystals were then recrystallized from methanol to obtain 1.56 g (yield 44%) of a light-yellow solid. The light-yellow solid was identified as 2-(4-dimethylaminophenyl)-4-(4-dimethylaminophenyl)-5-(2-fluorophenyl)-1,3-oxazole (the compound IX) from the following analysis results.

(1) Melting point 172°—173°C

(2) Elementary analysis values: $C_{25}H_{24}N_3OF$

| | C | H | N |
|---|---|---|---|
| Calculated (%) | 74.79 | 6.03 | 10.47 |
| Found (%) | 74.53 | 6.04 | 10.47 |

(3) NMR values (the NMR spectrum is shown in Fig. 3): $CDCl_3$

τ values

| | | |
|---|---|---|
| 2.06 | (doublet (2H) | aromatic ring |
| 2.40—2.90 | (multiplet 6H) | |
| 3.36 | (doublet 2H) | |
| 3.45 | (doublet 2H) | |

7.11 (singlet 12H the proton of a methyl group)

(4) IR spectrum (KBr method) is shown in Fig. 4.

## Example 3

In the dark, 32 g of concentrated sulfuric acid was maintained at 60°C and a mixture of 2.2 g of the 4-dimethylamino-2'-fluorobenzoin obtained in Synthesis Example 1 and 1.64 g of the 4-diethylaminobenzonitrile obtained in Synthesis Example 4 was added, after which the resulting mixture was stirred for 1 hour. When the reaction solution thus obtained was added to 800 ml of water, yellowish-green precipitate was deposited. After the precipitate was filtered, the filtrate was made basic with a 3N aqueous sodium hydroxide solution, upon which light-yellow crystals were deposited. The crystals were

filtered by means of suction and air-dried. The crystals were then recrystallized from methanol to obtain 1.39 g (yield 40%) of a light-yellow solid. The light-yellow solid was identified as 2-(4-diethylaminophenyl)-4-(4-dimethylaminophenyl)-5-(2-fluorophenyl)-1,3-oxazole (the compound X) from the following analysis results.

(1) Melting point 102°—105°C

(2) Elementary analysis values: $C_{27}H_{28}N_3OF$

| | C | H | N |
|---|---|---|---|
| Calculated (%) | 75.35 | 6.51 | 9.77 |
| Found (%) | 75.25 | 6.53 | 9.72 |

(3) NMR values (the NMR spectrum is shown in Fig. 5): $CDCl_3$

τ values

| | | |
|---|---|---|
| 2.09 | (doublet (2H) | aromatic ring |
| 2.40—2.95 | (multiplet 6H) | aromatic ring |
| 3.38 | (doublet 2H) | aromatic ring |
| 3.45 | (doublet 2H) | aromatic ring |

6.69 (quartet 4H the methylene proton of an ethyl group)

7.15 (singlet 6H the proton of a methyl group)

8.89 (triplet 6H the terminal methyl proton of an ethyl group)

(4) IR spectrum (KBr method) is shown in Fig. 6.

## Comparative Example 1

In the dark, 25 g of concentrated sulfuric acid was maintained at 60°C and a mixture of 1.2 g of 4-dimethylamino-2′-chlorobenzoin and 1.0 g of 4-diethylaminobenzonitrile was added, after which the resulting mixture was stirred for 1 hour and 30 minutes. When the reaction solution thus obtained was added to 500 ml of water, yellowish-green precipitate was deposited. After the precipitate was filtered, the filtrate was made basic with a 3N aqueous sodium hydroxide solution, upon which light-yellow crystals were deposited. The crystals were filtered by means of suction and air-dried. The crystals were then recrystallized from methanol to obtain 0.72 g (yield 37%) of a light-yellow solid. The light-yellow solid was identified as 2-(4-diethylaminophenyl)-4-(4-dimethylaminophenyl)-5-(2-chlorophenyl)-1,3 oxazole (the compound V) from the following analysis results.

(1) Melting point 135°—139°C

(2) Elementary analysis values: $C_{27}H_{28}N_3OCl$

| | C | H | N |
|---|---|---|---|
| Calculated (%) | 72.65 | 6.28 | 9.42 |
| Found (%) | 72.58 | 6.35 | 9.50 |

(3) NMR values (the NMR spectrum is shown in Fig. 7): $CDCl_3$

τ values

| | | |
|---|---|---|
| 2.05 | (doublet 2H) | aromatic ring |
| 2.45—2.82 | (multiplet 6H) | aromatic ring |
| 3.35 | (doublet 2H) | aromatic ring |
| 3.45 | (doublet 2H) | aromatic ring |

6.68 (quartet 4H the methylene proton of an ethyl group)

7.15 (singlet 6H the methyl proton of methyl group)

8.88 (triplet 6H the methyl proton of an ethyl group)

(4) IR spectrum (KBr method) is shown in Fig. 8.

## Test Example

Photolytic property tests on the compounds (VIII), (IX), (X) and (V) obtained in Example 1 to 3 and Comparative Example 1, respectively, were carried out in the following manner.

After nitogen gas was bubbled for 1 hour through acetronitrile solutions of each of the compounds (VIII), (IX), (X) and (V) having a concentration of $2 \times 10^{-4}$ mol/liter, each of the solutions was irridiated from a 150-W high pressure mercury arc lamp while bubbling therethrough nitrogen gas. Samples were collected at 5-minutes intervals after the beginning of the irridiation and diluted with acetonitrile in a definite dilution ratio, after which the change of the absorption spectrum was investigated by means of an ultraviolet spectrophotometer.

In the case of the compound (V) shown in Comparative Example, 5 minutes after the irradiation from the mercury arc lamp, the absorption spectrum changed to an absorption spectrum ($\lambda_{max}$ = 370.0 nm, 258.0 nm, 245.0 nm) which was different from the initial absorption spectrum ($\lambda_{max}$ = 363.0 nm $\varepsilon = 4.8 \times 10^4$ liter/mol.cm, 310 nm shoulder). The former absorption spectrum was due to 2-(4-diethylaminophenyl)-6-dimethylaminophenanthro[9,10-d]-oxazole produced by decomposition of the compound (V). The decomposition rate of the compound (V) shown in Comparative Example was calculated from the concentration of the decomposition product produced according to Lambert-Beer's law by measurement of the absorbance at 258.0 nm in the absorption spectrum at each time which absorbance appeared owing to the decomposition.

In the case of the compounds (VIII), (IX) and (X), even 60 minutes after the irradiation, the absorption spectra did not change in pattern from the initial spectrum (VIII: $\lambda_{max}$ = 363.0 nm $\varepsilon = 4.9 \times 10^4$ liter/mol.cm, 310.0 nm shoulder; IX: $\lambda_{max}$ = 359.0 nm $\varepsilon = 4.5 \times 10^4$ liter/mol.cm, 310.0 nm shoulder; X: $\lambda_{max}$ = 363.0 nm $\varepsilon = 4.45 \times 10^4$ liter/mol.cm, 310.0 nm shoulder). The decomposition rate of each of these compounds was evaluated from the ratio of the absorbance at 310.0 nm ($A_{310 \cdot 0}$) to the absorbance at $\lambda_{max}$ ($A\lambda_{max}$) ($A_{310 \cdot 0}/A\lambda_{max}$) in the absorption spectrum at each time.

In Fig. 9 are shown the changes with the lapse of time of the rates of photolysis of the acetonitrile solution of the compounds (VIII), (IX), (X) and (V) by the irradiation from the high pressure mercury arc lamp. In Fig. 9, the curve 1 shows the decomposition rate of the compound (VIII), the curve 2 that of the compound (IX), the curve 3 that of the compound (X), and the curve 4 that of the compound (V).

## Application Example 1

A 5% by weight solution of 1 part by weight of α type metal-free phthalocyanine (Heliogen Blue 7560, a trade name, manufactured by BASF A. G. and 6.7 parts by weight of a silicone resin (KR255, a trade name, manufactured by Shin-etsu Chemical Industry Co., Ltd.) in tetrahydrofuran as a solvent was prepared, and then kneaded in a ball mill for 5 hours. The pigment dispersion thus obtained was applied to an aluminum plate (an electric conductor) by means of an applicator and dried at 90°C for 40 minutes to form a charge generating material layer of 1 μm in thickness.

Next, a 27% by weight solution of 1 part by weight of each of the oxazole derivatives of this invention shown in Table 1, 2.4 parts by weight of a polyester resin (Vyron 200, a trade name, manufactured by Toyobo Co., Ltd.) and 0.6 part by weight of a styrene resin (Piccotex 100 manufactured by ESSO Standard Oil Company) in tetrahydrofuran as a solvent was prepared, applied to the above-mentioned charge generating material layer by means of an applicator, and then dried at 90°C for 40 minutes to form a charge transport material layer of 25 μm in thickness, whereby an electrophotographic plate was obtained.

The electrophotographic characteristics of the double-layer type electrophotographic plates thus produced were measured by means of an electrostatic recording paper analyzer (SP—428, a trade name, manufactured by Kawaguchi Electric Works Co., Ltd.). In Table 1 are shown the half decay exposure sensitivities measured by using a white lamp (a tungsten lamp) after corona electrical charging at negative 5 KV for 10 seconds followed by standing in the dark for 30 minutes.

Table 1

| Oxazole derivative | | Half decay exposure sensitivity |
|---|---|---|
| No. | Structural formula | (lux·sec.) |
| VIII | 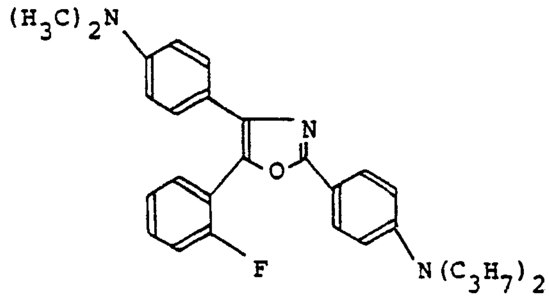 | 3.0 |
| IX | 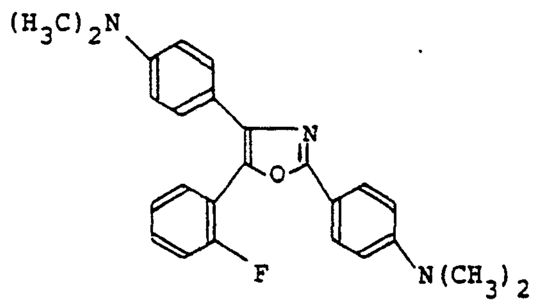 | 2.7 |
| X | 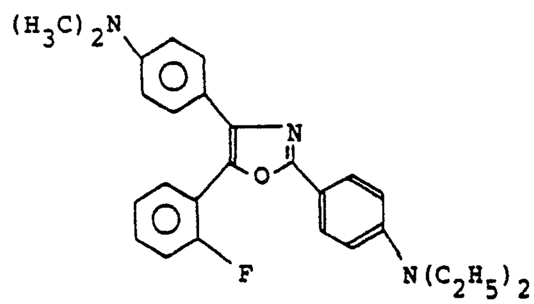 | 3.0 |

The oxazole derivative of this invention is so stable to ultraviolet light that it is not decomposed by irridiation therewith. Accordingly, at the time of use and when used for various purposes, said oxazole derivative is easy to handle and does not lose its characteristics. Further, its production is easy and therfore said oxazole derivative can be used as a photoconductive material, an electrochromium display material or the like.

**Claims**

1. An oxazole derivative of the formula:

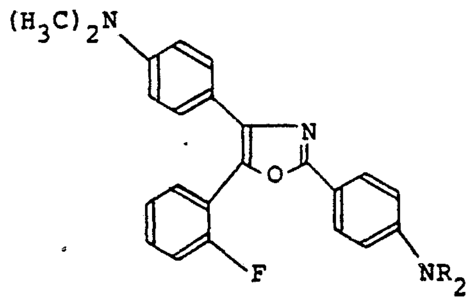 (I)

wherein R is an alkyl group having 1 to 3 carbon atoms.

2. An oxazole derivative according to Claim 1, which is 2-(4-dipropylaminophenyl)-4-(4-dimethylaminophenyl)-5-(2-fluorophenyl)-1,3-oxazole.

3. An oxazole derivative according to Claim 1, which is 2-(4-dimethylaminophenyl)-4-(4-dimethylaminophenyl)-5-(2-fluorophenyl)-1,3-oxazole.

4. An oxazole derivative according to Claim 1, which is 2-(4-diethylaminophenyl)-4-(4-dimethylaminophenyl)-5-(2-fluorophenyl)-1,3-oxazole.

5. A process for producing an oxazole derivative of the formula:

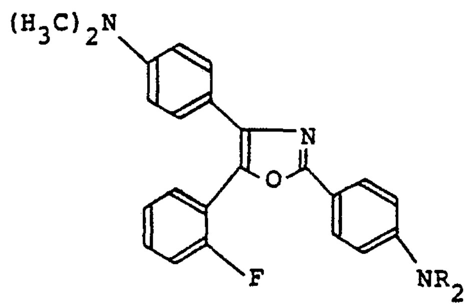 (I)

wherein R is an alkyl group having 1 to 3 carbon atoms, which comprises reacting a compound of the formula:

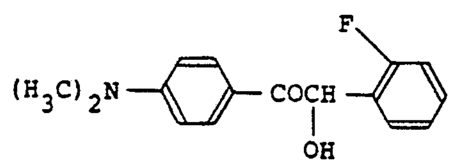 (II)

with a compound of the formula:

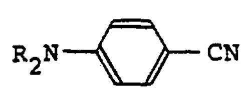 (III)

wherein R is as defined above.

6. A process according to Claim 5, wherein the reaction is carried out by dissolving both the compounds of the formulae (II) and (III) in concentrated sulfuric acid and maintaining in the dark at a temperature of 20°C to 70°C.

7. A process for using the oxazole derivative of the formula (I) as a photoconductive material.

8. A process according to Claim 7, wherein the photoconductive material is used as a charge transport material in an electrophotographic plate.

9. A process according to Claim 7, wherein the photoconductive material is used as a charge transport material in a charge transport material layer in a double-layer type electrophotographic plate.

**Patentansprüche**

1. Oxazolderivat der Formel

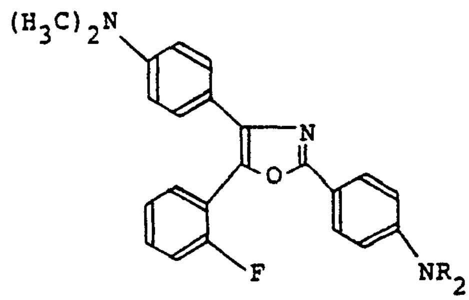

(I)

worin R eine Alkylgruppe mit 1 bis 3 Kohlenstoffatomen bedeutet.

2. Oxazolderivat nach Anspruch 1, nämlich 2-(4-Dipropylaminophenyl)-4-(4-dimethylaminophenyl)-5-(2-fluorphenyl)-1,3-oxazol.

3. Oxazolderivat nach Anspruch 1, nämlich 2-(4-Dimethylaminophenyl)-4-(4-dimethylaminophenyl)-5-(2-fluorphenyl)-1,3-oxazol.

4. Oxazolderivat nach Anspruch 1, nämlich 2-(4-Diethylaminophenyl)-4-(4-dimethylaminophenyl)-5-(2-fluorphenyl)-1,3-oxazol.

5. Verfahren zur Herstellung eines Oxazolderivats der Formel

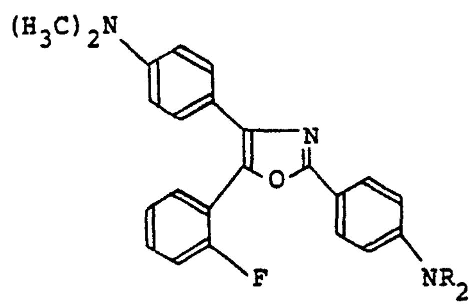

(I)

worin R eine Alkylgruppe mit 1 bis 3 Kohlenstoffatomen bedeutet, dadurch gekennzeichnet, daß man eine Verbindung der Formel

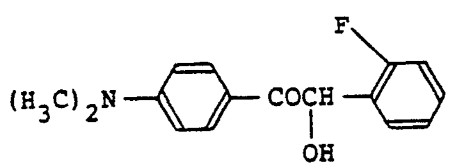

(II)

mit einer Verbindung der Formel

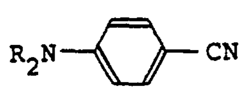

(III)

worin R die oben gegebene Definition besitzt, umsetzt.

6. Verfahren nach Anspruch 5, dadurch gekennzeichnet, daß die Reaktion durchgeführt wird, indem man beide Verbindungen der Formeln (II) und (III) in konzentrierter Schwefelsäure löst und sie in der Dunkelheit bei einer Temperatur von 20 bis 70°C stehen läßt.

7. Verfahren für die Verwendung eines Oxazolderivats der Formel (I) als photoleitendes Material.

8. Verfahren nach Anspruch 7, dadurch gekennzeichnet, daß das photoleitende Material als Ladungstransportmaterial in einer elektrophotographischen Platte verwendet wird.

9. Verfahren nach Anspruch 7, dadurch gekennzeichnet, daß das photoleitende Material als Ladungstransportmaterial in einer Ladungstransportmaterialschicht in einer doppelschichtigen elektrophotographischen Platte verwendet wird.

**Revendications**

1. Dérivé d'oxazole de formule (I) ci-après:

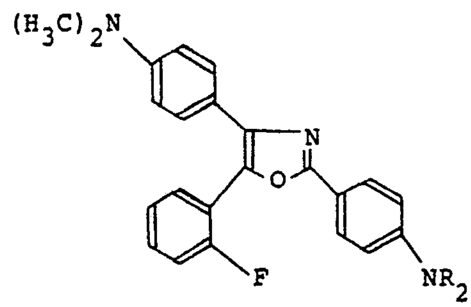

(I)

dans laquelle R représente un groupe alkyle contenant 1 à 3 atomes de carbone.

2. Dérivé d'oxazole selon la revendication 1, caractérisé en ce qu'il est le 2-(4-dipropylaminophényl)-4-(4-diméthylaminophényl)-5-(2-fluorophényl)-1,3-oxazole.

3. Dérivé d'oxazole selon la revendication 1, caractérisé en ce qu'il est le 2-(4-diméthylaminophényl)-4-(4-diméthylaminophényl)-5-(2-fluorophényl)-1,3-oxazole.

4. Dérivé d'oxazole selon la revendication 1, caractérisé en ce qu'il est le 2-(4-diéthylaminophényl)-4-(4-diméthylaminophényl)-5-(2-fluorophényl)-1,3-oxazole.

5. Procédé pour la préparation de dérivé d'oxazole de formule (I)

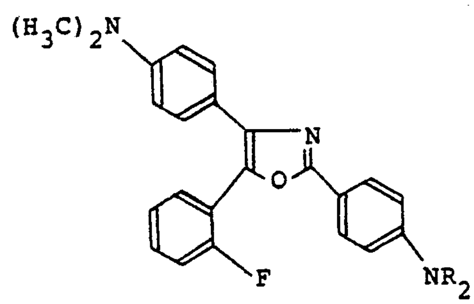

(I)

dans laquelle R représente un groupe alkyle contenant 1 à 3 atomes de carbone, caractérisé en ce que l'on fait réagir le composé de formule (II)

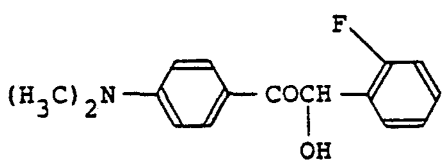

(II)

avec un composé de formule (III)

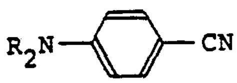

(III)

où R a la même signification que ci-dessus.

6. Procédé selon la revendication 5, caractérisé en ce que la réaction a lieu en dissolvant les deux composés de formules (II) et (III) dans l'acide sulfurique concentré et en maintenant dans l'obscurité et à une température comprise entre 20 et 70°C.

7. Procédé pour utiliser le dérivé d'oxazole de formule (I) en tant que matériau photoconducteur.

8. Procédé selon la revendication 7, caractérisé en ce que le matériau photoconducteur est utilisé en tant que matériau de transport de charge dans une plaque électrophotographique.

9. Procédé selon la revendication 7, caractérisé en ce que le matériau photoconducteur est utilisé en tant que matériau de transport de charge contenu dans la couche constituée de matériau transport de charge dans une plaque électrophotographique de type à double couches.

FIG. I

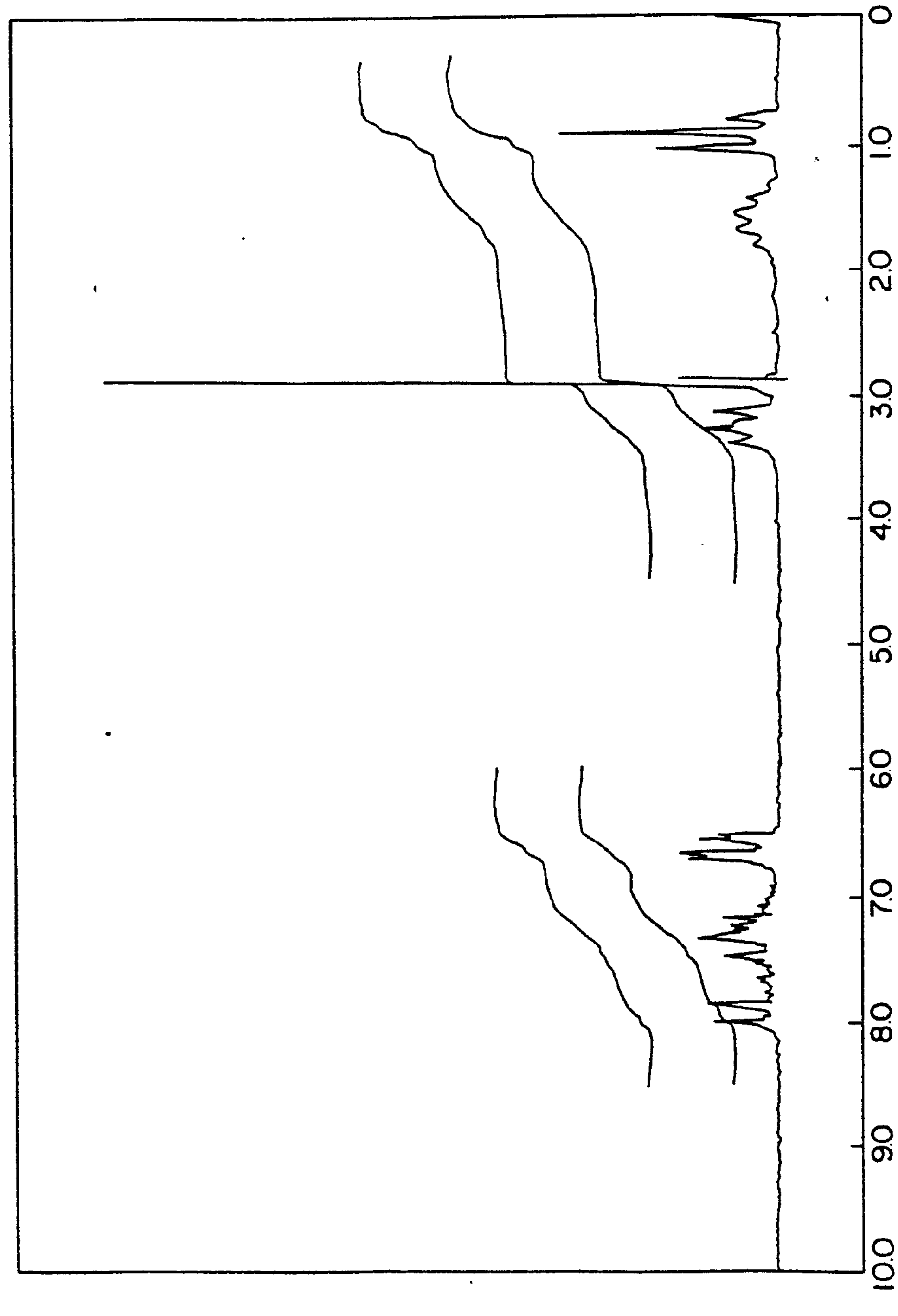

FIG. 2

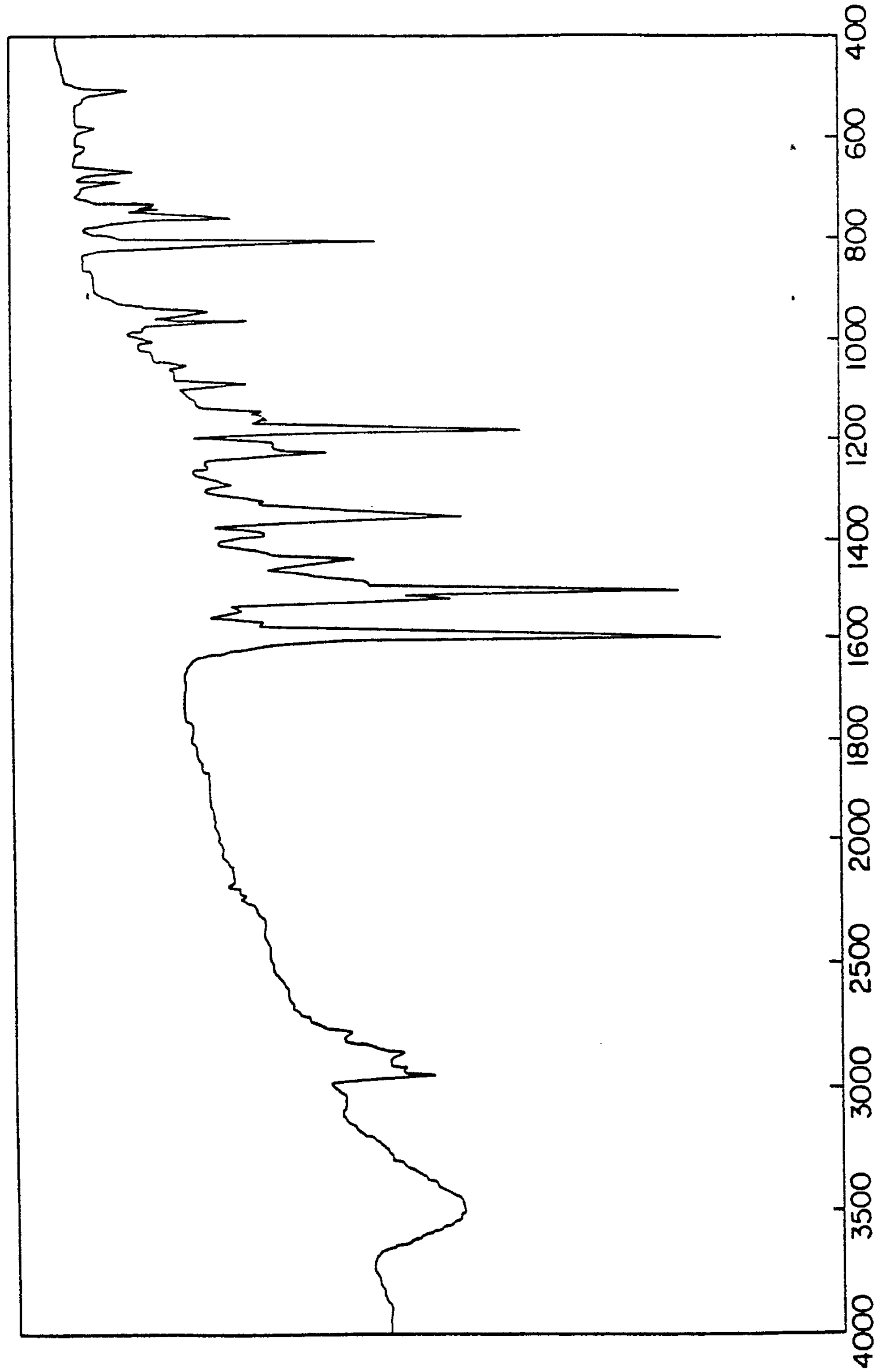

FIG. 3

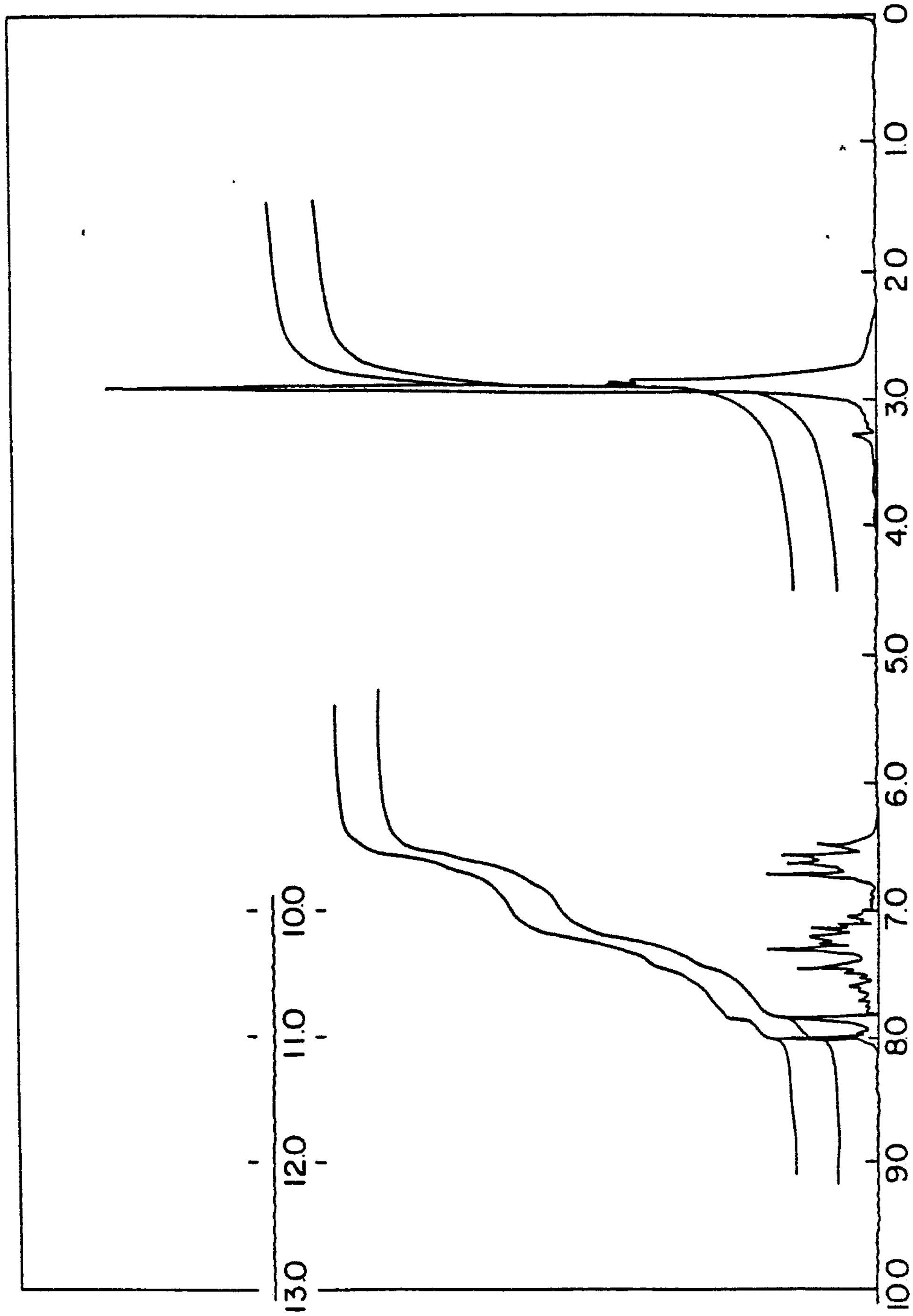

FIG. 4

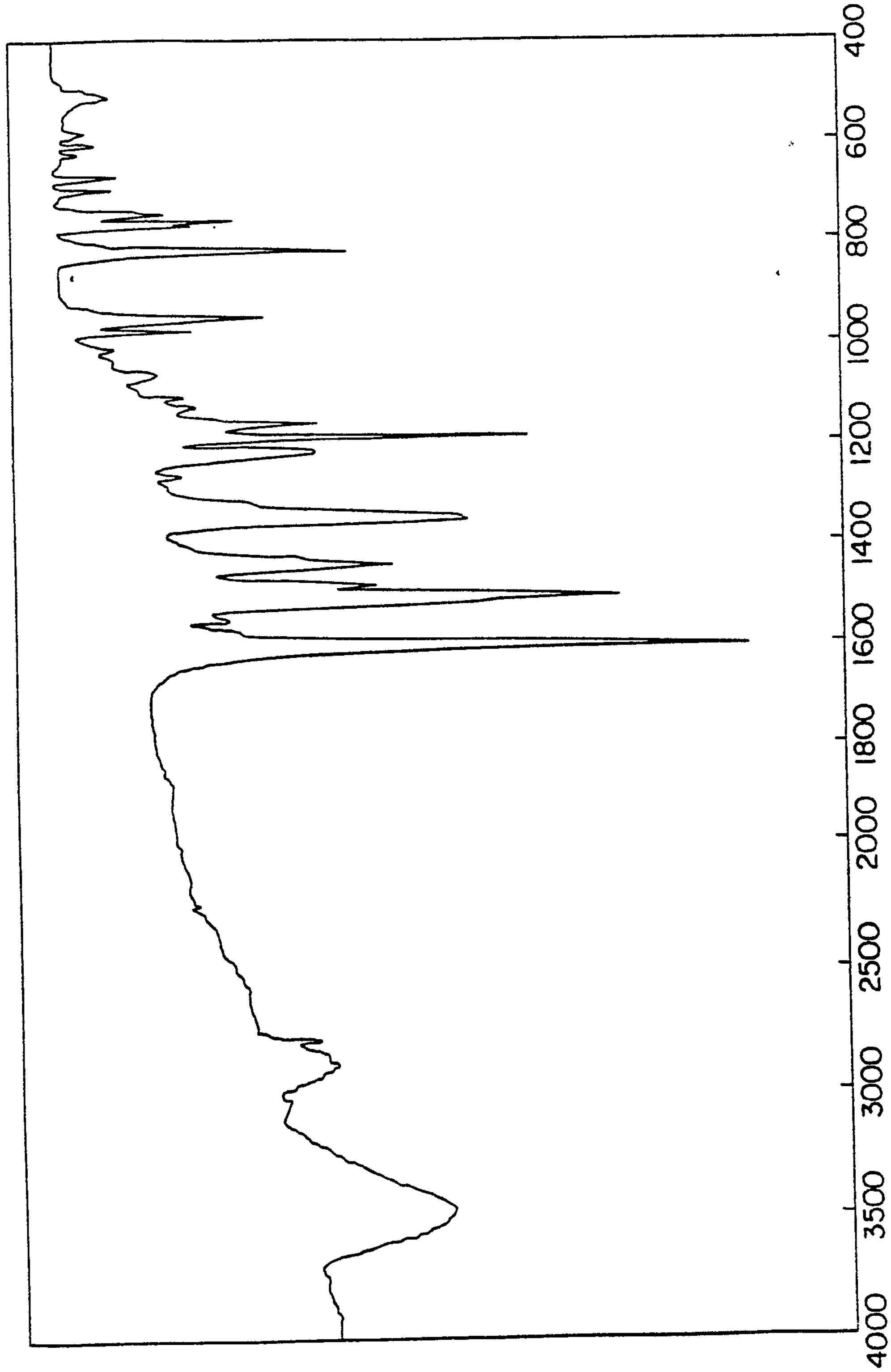

FIG. 5

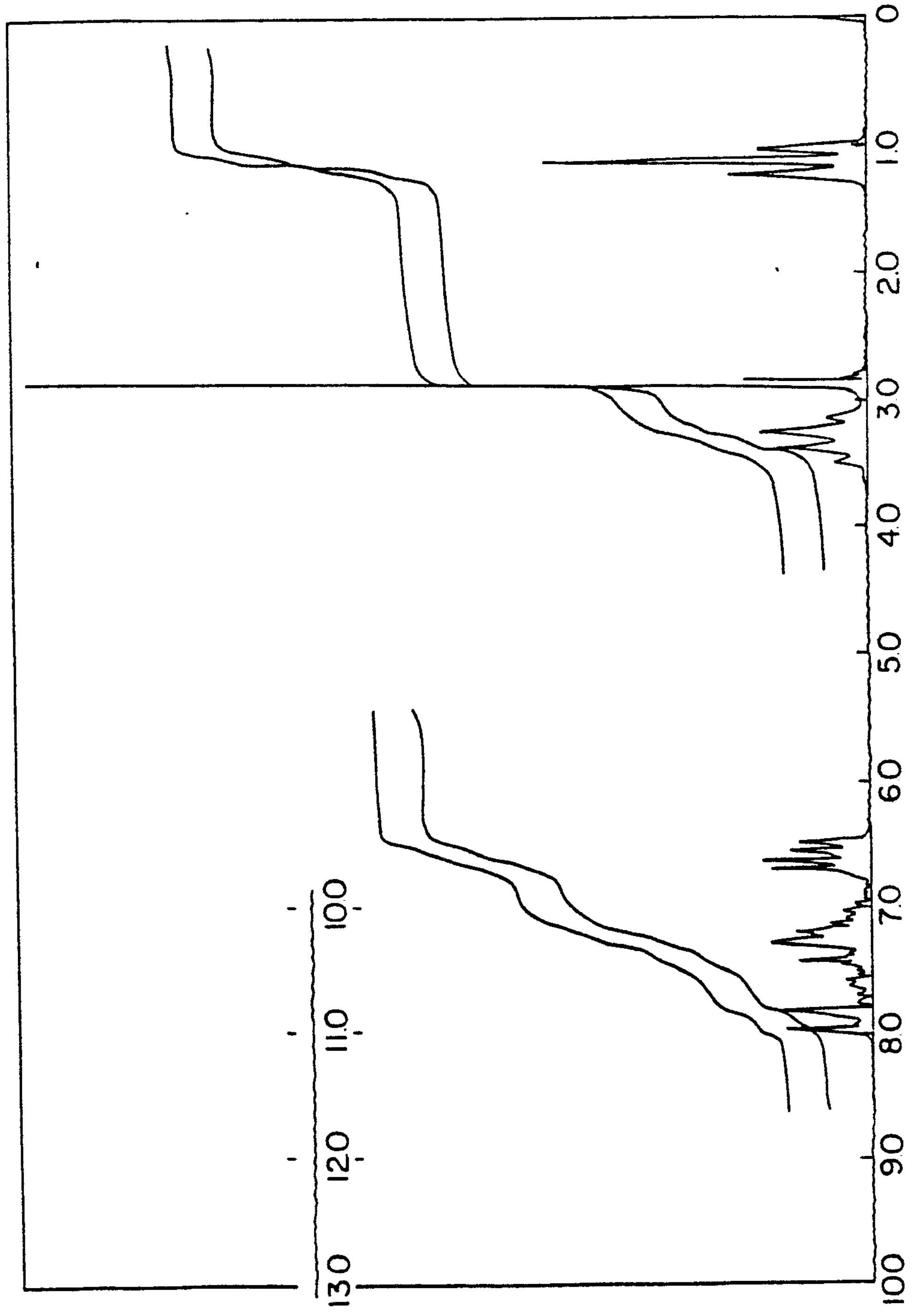

FIG. 6

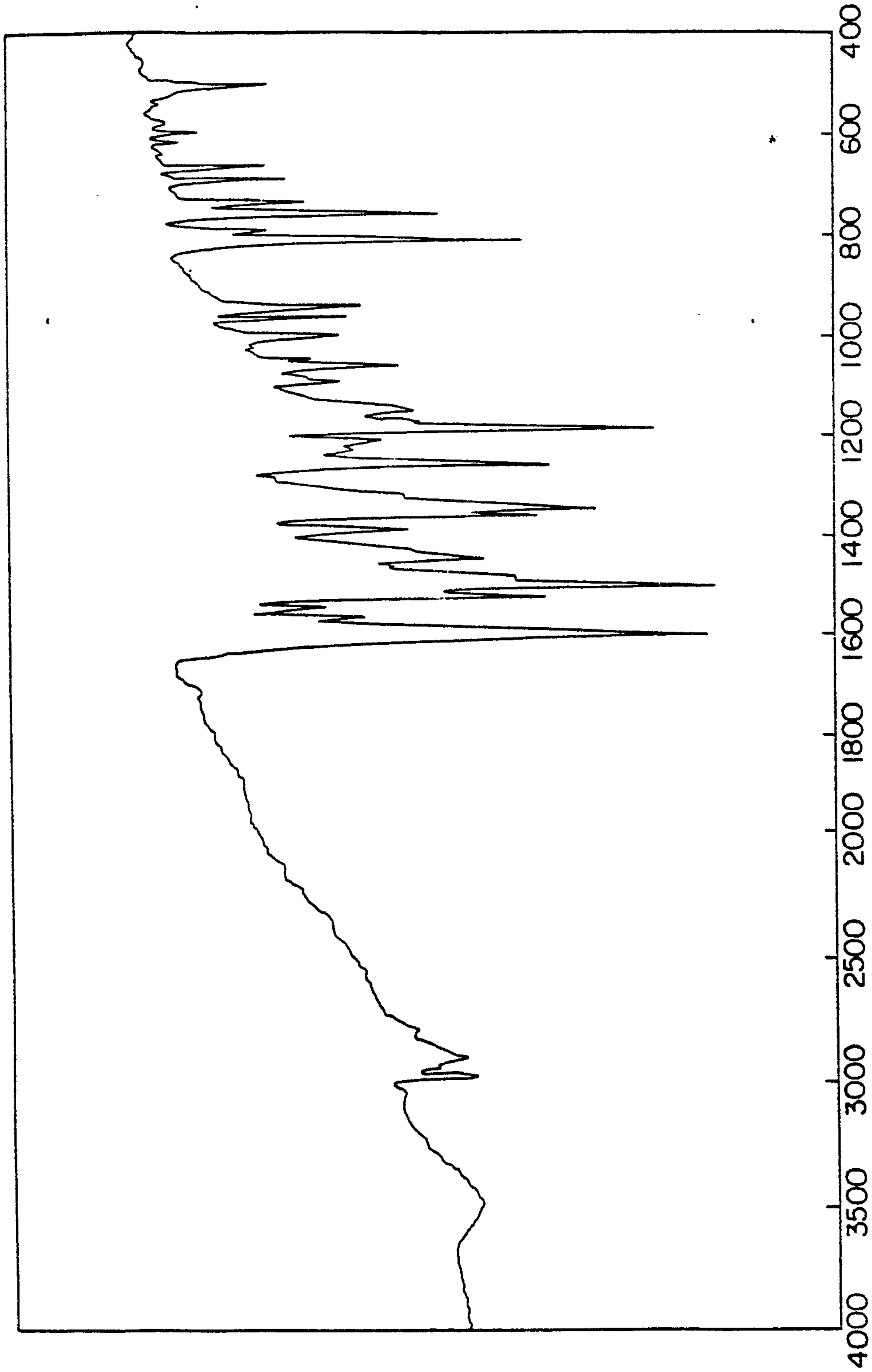

FIG. 7

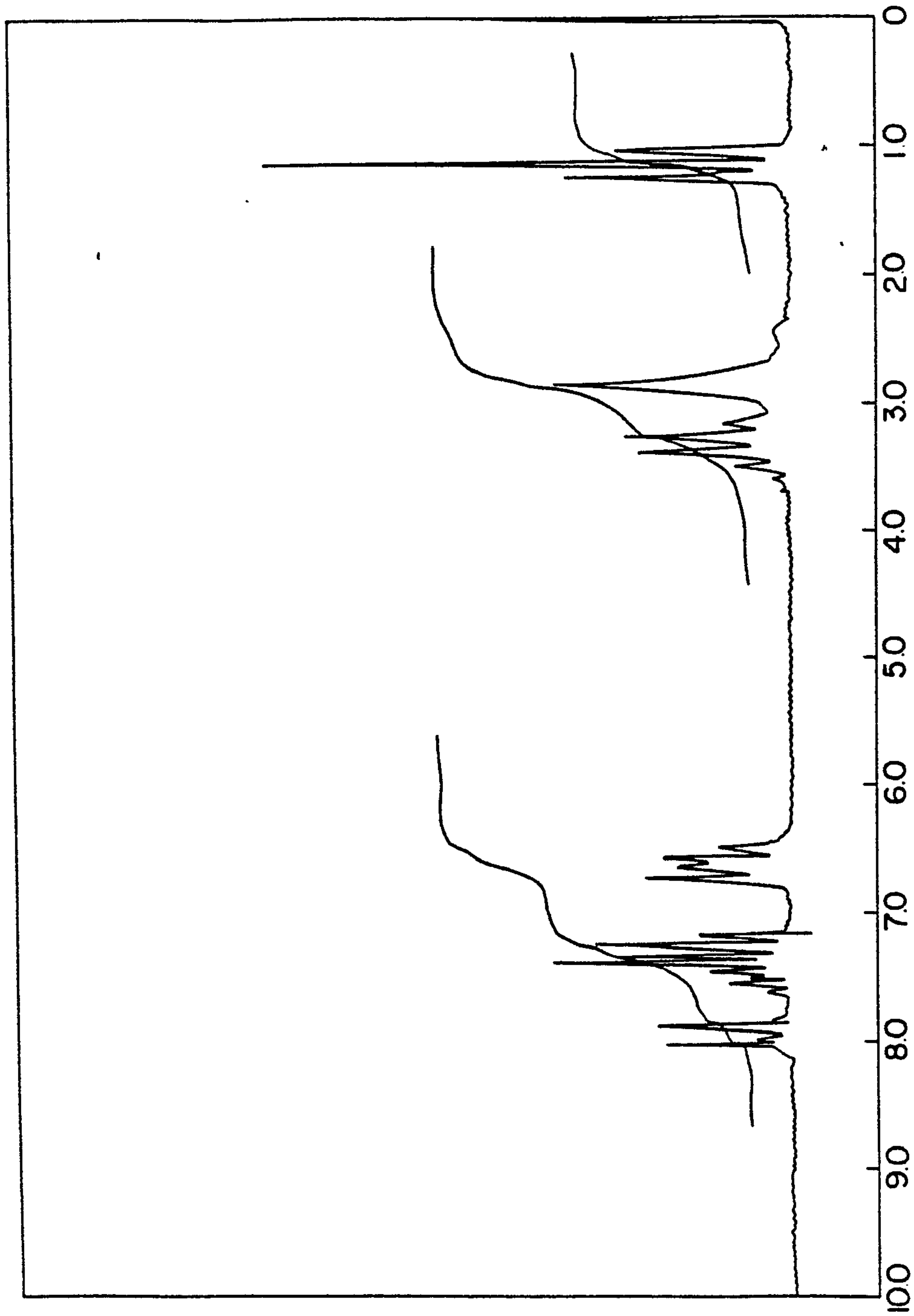

FIG. 8

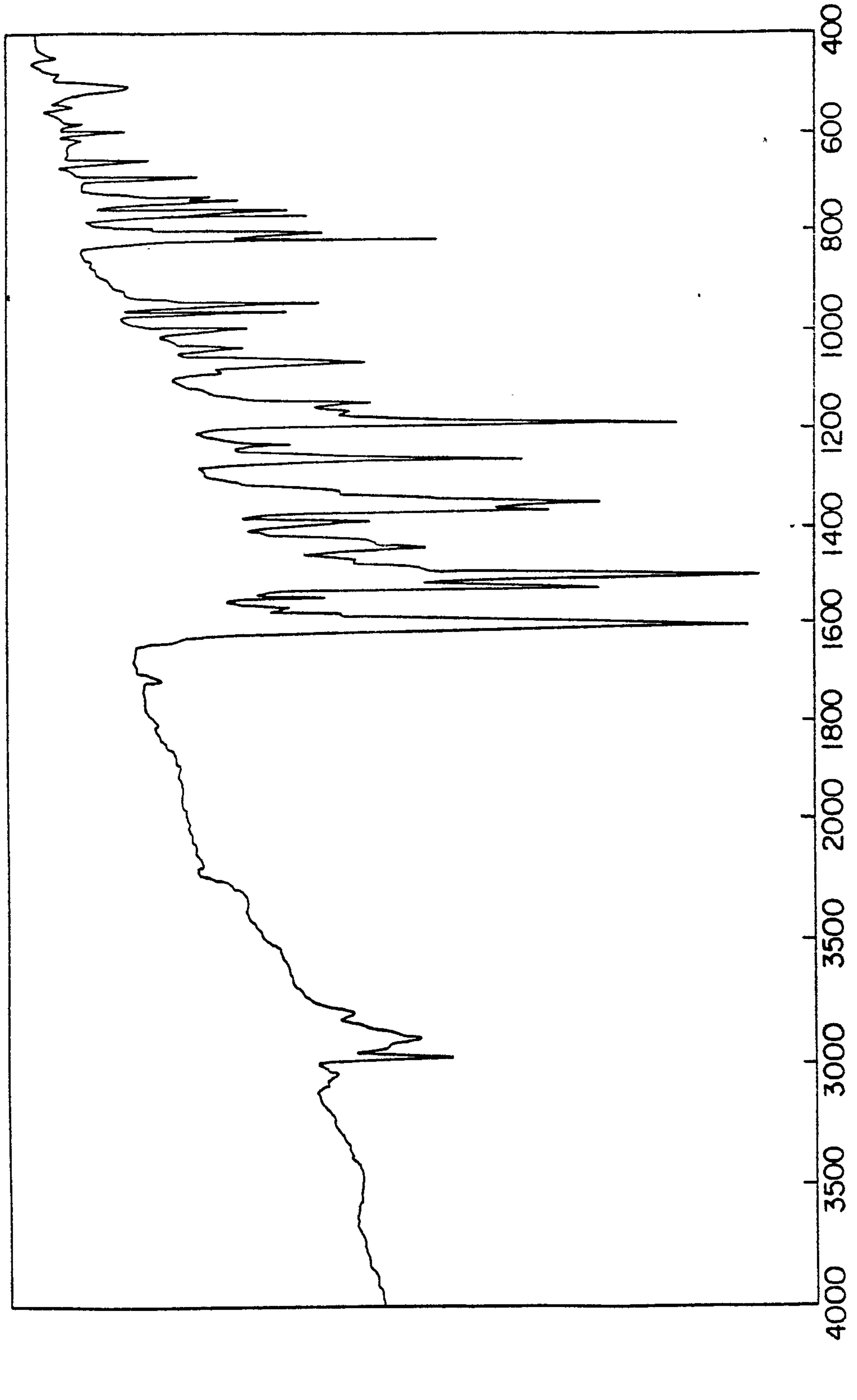

FIG. 9

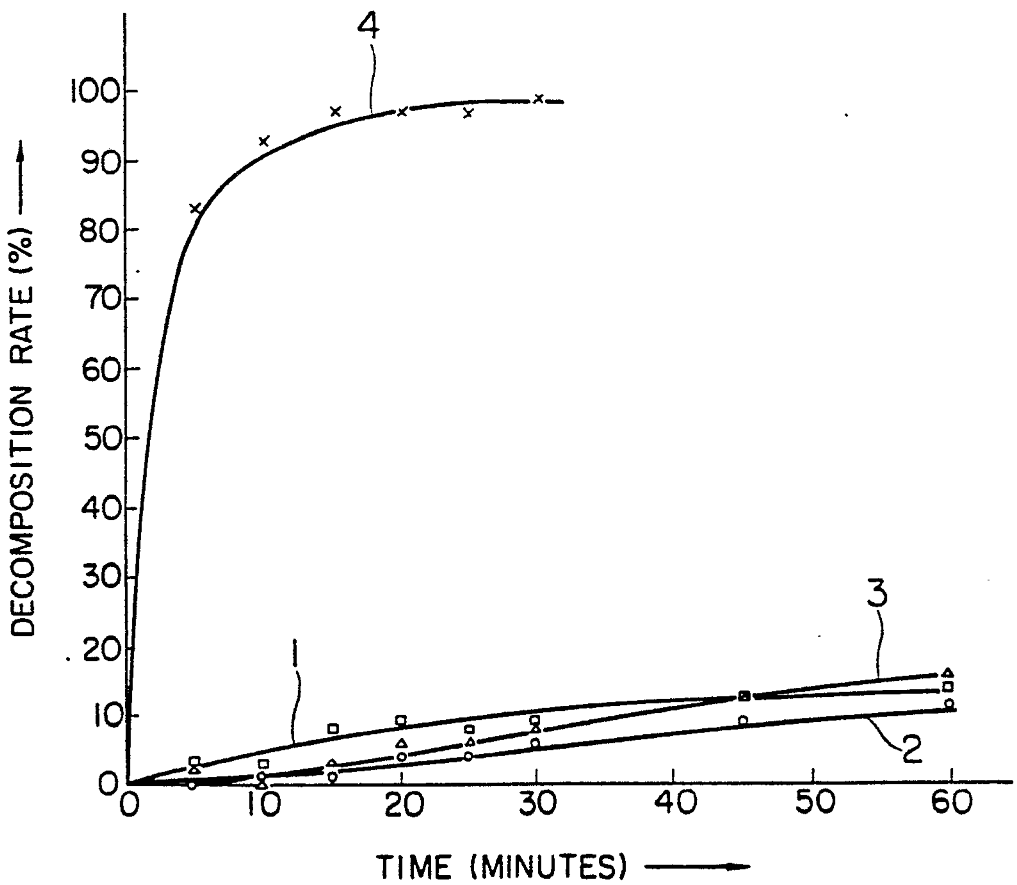

4
1
2
3
DECOMPOSITION RATE (%)
0
10
20
30
40
50
60
70
80
90
100
TIME (MINUTES)
0
10
20
30
40
50
60